# EUROPEAN PATENT APPLICATION

(11) **EP 1 703 315 A1**
(43) Date of publication of application: **20.09.2006**
(21) Application number: 06003884.1
(22) Date of filing: 25.02.2006
(51) Int. Cl.: G02F 1/1333, G02F 1/1339, G02F 1/1341

(54) **Phase-separation high molecular weight polymer adhesive structures in a flexible liquid crystal display**

(30) Priority: 15.03.2005 KR 2005021405
(71) Applicant: Samsung Electronics Co., Ltd., Yeongtong-gu Suwon-city, Gyeonggi-do 442-742 (KR); Industry University Cooperation Foundation, Seoul 133-791 (KR)
(72) Inventor: Kim, Jae-Hoon, Jukjeon-dong Yongin-si Gyeonggi-do (KR)
(74) Representative: Dr. Weitzel & Partner

(57) **Abstract**

A liquid crystal display (LCD) includes a first insulating substrate (210), a second insulating substrate (110) arranged opposite to the first insulating substrate, a liquid crystal layer (3) arranged between the first insulating substrate and the second insulating substrate, a spacer (321,322) which defines and maintains a substantially uniform gap between the first insulating substrate and the second insulating substrate, and a high molecular weight layer (331) which is provided on a surface of the second insulating substrate and attaches the spacer to the second insulating substrate.
The highmolecular weight polymer layer (331) is formed by phase separation from a mixture of precursor (monomer) and liquid crystal solution under UV irradiation. The polymer binds the spacer (321) to the substrate to finish the cell. The method of adhering the substrate is particularly applicable to flexible LCDs having plastic substrates. The spacer (321) divides the display into a lattice of cells or stripes. In an alternative embodiment the spacer consists of parallel walls (322) and the cells are finished by exposing the polymer precursor/LC mixture perpendicularly to the spacer walls (332).

## Description

### CROSS REFERENCE TO RELATED APPLICATION

This application claims priority to and the benefit of Korean Patent Application No. 10-2005-0021405 filed on March 15, 2005, which is hereby incorporated by reference for all purposes as if fully set forth herein.

### BACKGROUND OF THE INVENTION

### Field of the Invention

The invention relates to a liquid crystal display and a method of manufacturing the same, and more particularly, to a flexible liquid crystal display and a method of manufacturing the same.

### Description of the Related Art

An LCD includes a pair of panels individually having electrodes on their inner surfaces, and a dielectric anisotropy liquid crystal layer arranged between the panels. In the LCD, a voltage difference between the field generating electrodes, i.e., a variation in the strength of an electric field generated by the electrodes, is varied to change the transmittance of a light passing through the LCD. Desired images are obtained by controlling the voltage difference between the electrodes. The light may be a natural light or an artificial light emitted from a light source that is separately used in the LCD.

The liquid crystal layer is formed in a cell gap located between the two panels. For good image display, it is important to form and maintain the cell gap uniformly.

Flexible LCDs that are capable of bending, e.g., like a paper, are being researched and developed, specifically a flexible LCD having sufficiently thin substrates composed of pliable plastic instead of rigid glass.

However, it is difficult to maintain the cell gap uniformly when the flexible display is in a flexible state, e.g., folded or rolled up. If a uniform cell gap is not maintained, the required image display cannot be displayed and the image becomes distorted.

### SUMMARY OF THE INVENTION

The invention provides a technology for forming and maintaining a uniform cell gap between a pair of panels of an LCD and for tightly assembling the panels.

Additional features of the invention will be set forth in the description which follows, and in part will be apparent from the description, or may be learned by practice of the invention.

The present invention discloses a liquid crystal display (LCD), including a first insulating substrate; a second insulating substrate arranged opposite to the first insulating substrate; a liquid crystal layer arranged between the first insulating substrate and the second insulating substrate; a spacer that forms and maintains a substantially uniform gap between the first insulating substrate and the second insulating substrate; and a high molecular weight layer formed on a surface of the second insulating substrate that connects the spacer with the second insulating substrate.

The present invention also discloses a liquid crystal display (LCD), including a first insulating substrate; a second insulating substrate arranged opposite to the first insulating substrate; a liquid crystal layer arranged between the first insulating substrate and the second insulating substrate; a spacer that forms and maintains a substantially uniform gap between the first insulating substrate and the second insulating substrate; and a high molecular weight projection that is arranged between the first insulating substrate and the second insulating substrate.

The present invention also discloses a method of manufacturing a liquid crystal display, including forming a first electrode on a first insulating substrate; forming a spacer on the first electrode; applying a mixture of a polymerizable monomer and liquid crystalline material on the first electrode with the fixed spacer; arranging a second insulating substrate on the fixed spacer; phase-separating the polymerizable monomer from the liquid crystalline material during polymerization; and attaching the phase-separated polymer to a surface of the second insulating substrate such that the spacer and the second insulating substrate are attached thereto.

It is to be understood that both the foregoing general description and the following detailed description are exemplary and explanatory and are intended to provide further explanation of the invention as claimed.

### BRIEF DESCRIPTION OF THE DRAWINGS

The accompanying drawings, which are included to provide a further understanding of the invention and are incorporated in and constitute a part of this specification, illustrate embodiments of the invention and together with the description serve to explain the principles of the invention.

Fig. 1 is a perspective view of an LCD according to a first embodiment of the invention.

Fig. 2 is a perspective view showing the state of a spacer arranged on a common electrode panel used in the LCD shown in Fig. 1.

Fig. 3 is a cross-sectional view of the LCD shown in Fig. I.

Fig. 4 is a top-front view of a spacer used in an LCD according to the first embodiment of the invention.

Fig. 5 is a perspective view of the spacer shown in Fig. 4.

Fig. 6 is a photograph of an LCD according to the first embodiment of the invention while in a black state.

Fig. 7 is a photograph of an LCD according to the first embodiment of the invention while in a white state.

Fig. 8 is a graph showing a correlation between voltage (V) and transmittance (T) in an LCD according to the first embodiment of the invention.

Fig. 9 is a graph showing the response time of an LCD according to the first embodiment of the invention.

Fig. 10 and Fig. 11 are photographs taken after two insulating substrates used in an LCD according to the first embodiment of the invention are separated from each other.

Fig. 12 is a perspective view of an LCD according to a second embodiment of the invention.

Fig. 13 is a perspective view showing the state of a spacer formed on a common electrode panel used in the LCD shown in Fig. 12.

Fig. 14 illustrates a UV irradiation process used to produce an LCD according to the second embodiment of the invention.

Fig. 15 is a photograph taken when the LCD shown in Fig. 12 is in a white state.

Fig. 16 is a photograph taken when the LCD shown in Fig. 12 is in a black state.

Fig. 17 and Fig. 18 are photograph taken after two insulating substrates used in an LCD according to the second embodiment of the invention are separated from each other.

Fig. 19A, 19B, 19C, 19D, and 19E are schematic cross-sectional views showing operations to manufacture an LCD according to the second embodiment of the invention.

Fig. 20 shows a SEM photograph of a stamp applicable to the operations shown in Fig. 19A, 19B, 19C, 19D, and Fig. 19E.

Fig. 21 shows a SEM photograph of a spacer formed using the stamp shown in Fig. 20.

Fig. 22 is an enlarged cross-sectional view of a portion represented as a circle in Fig. 21.

Fig. 23A and Fig. 23B are photographs taken when the LCD, manufactured through the operations shown in Fig. 19A, 19B, 19C, 19D, and 19E, is in a black state and a white state, respectively.

Fig. 23C and Fig. 23D are photographs taken when the LCD, manufactured through the operations shown in Fig. 19A, 19B, 19C, 19D, and 19E, is in medium gray states in response to gray voltages of 3V and 6V.

Fig. 24 is a perspective view of an LCD according to a third embodiment of the invention.

### DESCRIPTION OF THE ILLUSTRATED EMBODIMENTS

The invention is described more fully hereinafter with reference to the accompanying drawings, in which embodiments of the invention are shown. This invention may, however, be embodied in many different forms and should not be construed as limited to the embodiments set forth herein. Rather, these embodiments are provided so that this disclosure is thorough, and will fully convey the scope of the invention to those skilled in the art. In the drawings, the size and relative sizes of layers and regions may be exaggerated for clarity.

It is understood that when an element or layer is referred to as being "on" or "connected to" or "connected with" another element or layer, it can be directly on or directly connected to or with the other element or layer or intervening elements or layers may be present. It is also understood that when an element such as a layer, film, region, or substrate is referred to as being "on" another element, it can be directly on the other element or intervening elements may also be present.

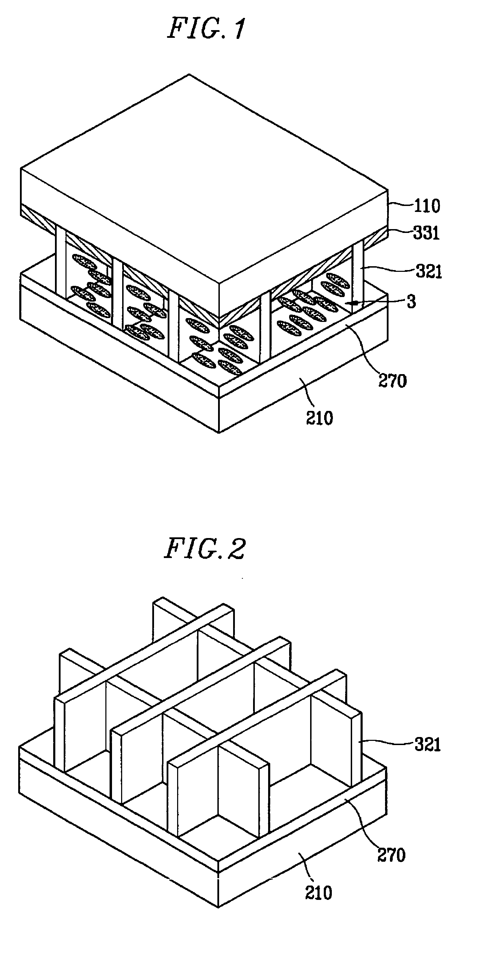

Hereinafter, an LCD according to a first embodiment of the invention is described with reference to Fig. 1 and Fig. 2.

Fig. 1 is a perspective view of an LCD and Fig. 2 is a perspective view showing the state of a spacer formed on a common electrode panel used in the LCD shown in Fig. 1.

Referring to Fig. 1, a spacer 321, e.g., lattice-shaped, is arranged between an upper insulating substrate 110 and a lower insulating substrate 210 with a common electrode 270. A high-molecular weight layer 331 is arranged on an inner surface of the upper substrate 110.

The spacer 321 maintains a space (referred to as a cell gap) between the two substrates 110 and 210, and partitions the space into a plurality of regions. As shown in Fig. 1, each region may correspond to a pixel unit or a combination of pixel units. The regions are filled with nematic liquid crystal 3.

The spacer 321 may be formed using a photolithography process. For example, after the lower substrate 210 is coated with a photoresist, the photoresist-coated substrate 210 is selectively exposed to UV light via a predetermined photo-mask and the exposed photoresist is then developed, resulting in the formation of the spacer 321. The spacer 321 is not limited to being made of a photoresist material and may be made of other materials such as an elastic polymer, etc. Such materials are described below.

The upper substrate 110 and the spacer 321 are attached with, e.g., adhered to, the high molecular weight layer 331. This high molecular weight layer 331 may be formed by polymerizing a monomer through UV irradiation via a mask. For example, the monomer, such as Norland optical adhesive (NOA-65) which is a product of Norland Co., is mixed with the liquid crystal 3, and the mixture is inserted, e.g., injected, into the spaces formed by the spacer 321. After the insertion, the upper substrate 110 is disposed on the spacer 321, and UV light is irradiated upon the upper substrate 110 via a mask. The monomer polymerizes with the UV irradiation while being anisotropically phase-separated from the liquid crystal 3. The phase-separated polymer then becomes attached with the inner surface of the upper substrate 110, thereby forming the high molecular weight layer 331.

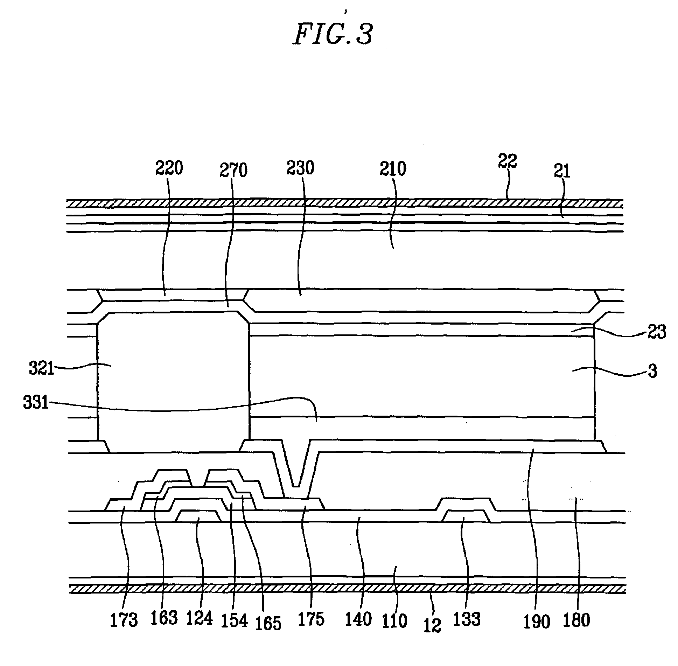

The above-described LCD is described below with reference to Fig. 3.

Fig. 3 is a cross-sectional view after the LCD of Fig. 1 is reversed above and below such that the lower insulating substrate 210 is shown on the top and the upper insulating substrate 110 is shown on the bottom.

Referring to Fig. 3, a gate electrode 124 and a storage electrode 133 are arranged on an inner surface of the upper substrate 110, e.g., facing the lower substrate 210. The gate electrode 124, which is a partial portion of a gate line (not shown), supplies a scanning signal. The storage electrode 133, which is a partial portion of a storage electrode line (not shown), receives a predetermined voltage to form a storage capacitance.

A gate insulating layer 140 is arranged on the gate electrode 124 and the storage electrode 133.

An amorphous silicon (a-Si) pattern 154 is formed on the gate insulating layer 140, and ohmic contacts 163 and 165 are formed on the a-Si pattern 154, while being separated from each other.

A source electrode 173 connected, e.g., coupled, with a data line (not shown) and a drain electrode 175 are formed on the ohmic contacts 613 and 165, respectively, while being separated from and facing each other.

A passivation layer 180 is arranged on the source electrode 173 and the drain electrode 175. The passivation layer 180 may be made of an inorganic insulator material such as silicon nitride and silicon oxide, an organic insulator such as resin, or a low dielectric insulator such as a-Si:C:O and a-Si:O:F that may be formed by a plasma enhanced chemical vapor deposition (PECVD).

The passivation layer 180 includes a contact hole that exposes a portion of the drain electrode 175 therethrough. A pixel electrode190 is formed on the passivation layer 180 and is connected, e.g., coupled, with the drain electrode 175 through the contact hole. The pixel electrode 190 may be made of a transparent material such as indium tin oxide (ITO) and indium zinc oxide (IZO). In reflective-type LCD_{S}, the pixel electrode 190 may be made of a conductive material having prominent light-reflecting characteristics, such as aluminum (Al) or the like.

A black matrix 220 is provided on an inner surface of the lower substrate 210, e.g., e.g., facing the upper substrate 110, to prevent light from leaking out through barriers between the pixels. Red, green, and blue (RGB) color filters 230 may be individually provided at pixel regions that are partitioned by the black matrix 220. Additionally, a white color filter, through which white light passes intact, may be provided and/or yellow, cyan, and magenta color filters may replace be used to complement the RGB color filters 230.

A common electrode 270, which may be made of ITO or IZO, is formed on the color filters 230.

An alignment layer 23 is formed on the common electrode 270 to align liquid crystal molecules in a predetermined direction.

A spacer 321 is provided between the upper substrate 110 and lower substrate 210. This is tightly attached to the passivation layer 180 and the pixel electrodes 190 of the upper substrate 110 by the high molecular weight layer 331.

Liquid crystal is inserted or filled in a cell gap located between the two substrates 110 and 210, the cell gap being maintained by the spacer 321, thereby forming a liquid crystal layer.

As shown in Fig. 3, a first polarizing plate 12 may be arranged on an outer surface of the upper substrate 110, and a retardation film 21 and a second polarizing plate 22 may be arranged on an outer surface of the lower substrate 210. It is understood that the retardation film 21 may be omitted or a plural number of retardation films 21 may be arranged on the lower substrate 210. In addition, another retardation film may be arranged between the first polarizing plate 12 and the upper substrate 110.

A method for manufacturing the above-described LCD is discussed below.

A metallic material, e.g., metal, is deposited on the upper insulating substrate 110 and photoetching is then performed on the metallic material to form gate lines with gate electrodes 124 and storage electrode lines with storage electrodes 133.

A gate insulating layer 140, an a-Si layer, and an a-Si layer doped with N-type impurities are successively deposited on the gate lines with the gate electrodes 124 and the storage electrode lines with the storage electrodes 133.

The a-Si layer and the doped a-Si layer are photoetched at the same time to form ohmic contacts 163 and 165 in the incomplete state, which are not separated from the a-Si pattern 154.

A metallic layer is then deposited on the incomplete ohmic contacts 163 and 165 and a photoetching process is performed on the deposited metallic layer to form data lines with source electrodes 173 and drain electrodes 175.

The ohmic contacts 163 and 165 are then completely formed by selectively etching the ohmic contacts 163 and 165 using a mask of the source electrodes 173 and the drain electrodes 175, so that the ohmic contacts 163 and 165 are separated from each other.

A passivation layer 180 is then deposited on the data lines and the drain electrodes 175, and a photoetching process is then performed on the deposited passivation layer 180 to form contact holes that expose the drain electrodes 175.

After forming the contact holes, a transparent material such as ITO or IZO may be deposited on the passivation layer 180 and a photoetching process may be performed on the deposited transparent material, thereby forming pixel electrodes 190.

The operations for forming the color filters 230 on the lower insulating substrate 210 are described below according to an embodiment of the invention.

A single layer of chrome or a double layer of chrome and chrome oxide may be deposited on the lower substrate 210 and a photoetching process is then performed on the single layer of deposited chrome or the double layers of deposited chrome and chrome oxide, thereby forming a black matrix 220 that partitions a display panel into pixel regions or pixel matrices.

Subsequently, the black matrix 220 may be coated with photoresist that includes pigments. Then, UV irradiation and UV developing processes are successively performed. Such processes are repeatedly performed to form red, green, and blue (RGB) color filters 230.

A transparent material such as ITO or IZO is then deposited on the color filters 230, thereby forming a common electrode 270.

A method for assembling the lower substrate 210 and the upper substrate 110 is described below according to an embodiment of the invention.

A photoresist is coated or applied on the common electrode 270 of the lower substrate 210, and an exposure process using a predetermined mask and a developing process are successively performed on the coated photoresist, thereby forming a spacer 321.

The spacer 321 is then coated or applied with an aligning agent and a rubbing process is performed thereon, thereby forming an alignment layer 23.

A monomer that can be polymerized by UV radiation is then combined with the liquid crystal. For example, the monomer may be UV-curing epoxy NOA-65, which is produced by Norland Co. The mixture is then filled or inserted in the regions partitioned by the spacer 321.

The upper substrate 110 having the TFTs and the pixel electrodes 190 formed thereon is then arranged the spacer 321 and UV light is irradiated thereto. At this time, the UV-irradiated monomer begins to polymerize while being phase-separated from the liquid crystal. The phase-separated polymer is attached with the surfaces of the pixel electrodes 190 and the passivation layer 180 of the upper substrate 110, resulting in the formation of a high molecular weight layer 331.

The high molecular weight layer 331 operates as a sufficient adhesive between the spacer 321 and the upper substrate 110.

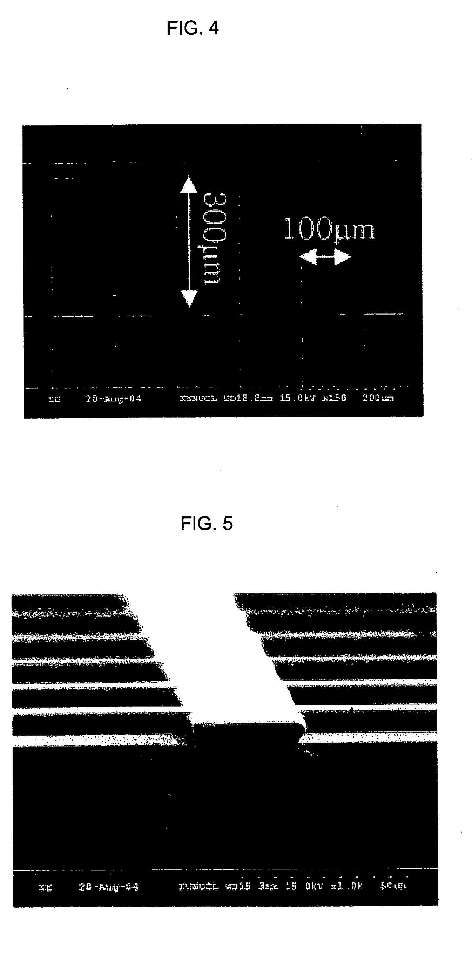

Fig. 4 is a top-front view of a spacer used in an LCD according to the first embodiment of the invention, and Fig. 5 is a perspective view of the spacer shown in Fig. 4.

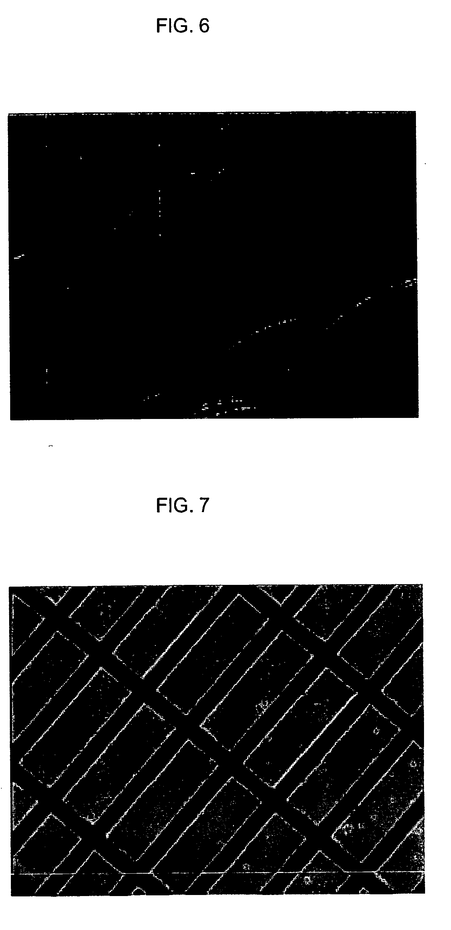

As shown in Fig. 4 and Fig. 5, the first embodiment of the present invention includes a lattice-shaped spacer wherein each of the regions partitioned by the spacer has a size of approximately 100 µm X 300 µm. Photographs taken when an electric field is generated in the LCD incorporating such a spacer are shown in Fig. 6 and Fig. 7, respectively.

Fig. 6 is a photograph taken when the LCD according to the first embodiment of the invention is in a black state. Fig. 7 is a photograph taken when the LCD according to the first embodiment of the invention is in a white state.

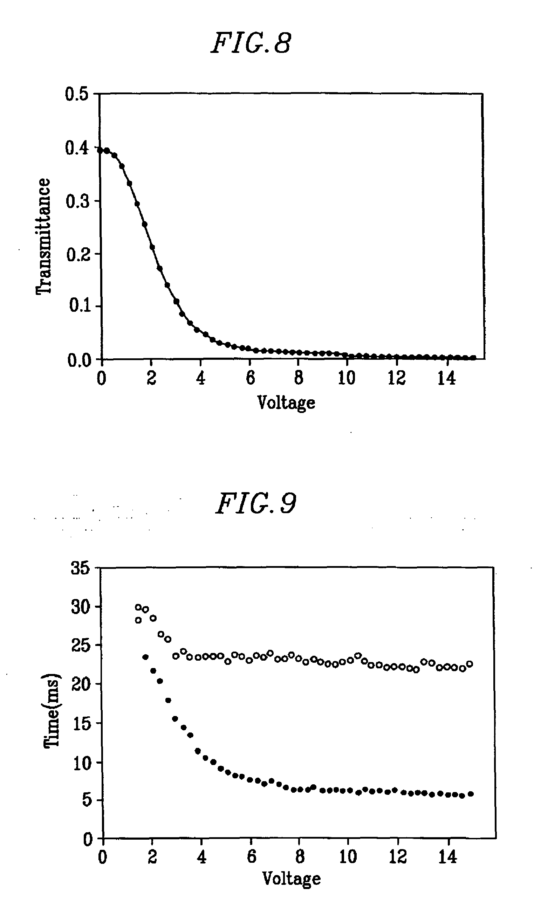

Fig. 8 is a graph showing a correlation between voltage (V) and transmittance (T) in an LCD according to the first embodiment of the invention.

In the graph of Fig. 8, typical V-T features of a normal white mode LCD are shown. As shown in Fig. 8, the transmittance is maximized when the voltage is 0V and the transmittance decreases as the voltage increases.

Fig. 9 is a graph showing the response time of an LCD according to the first embodiment of the invention.

Referring to Fig. 9, black dots represent response speed during the voltage-on state and white dots represent response speed during the voltage-off state. As shown in this graph, during both the voltage-on state and .the voltage-off state, the response speed is less than 30ms. Accordingly, this LCD may effectively display moving images.

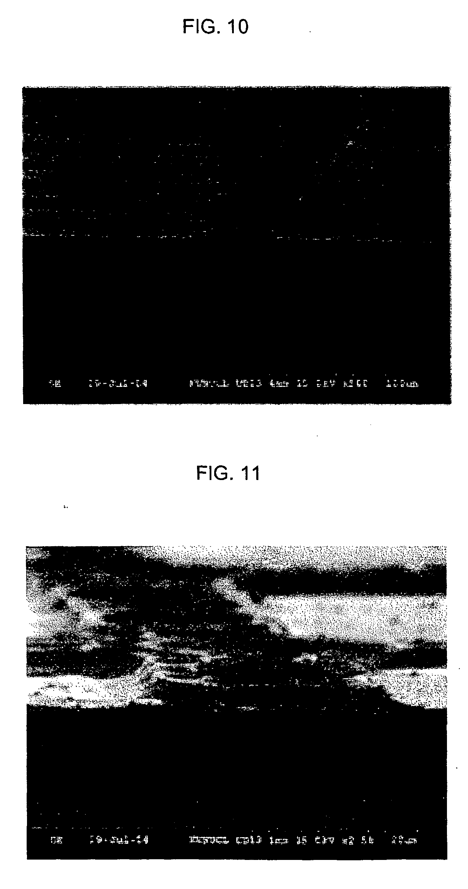

Fig. 10 and Fig. 11 are photographs taken after two insulating substrates used in an LCD according to the first embodiment of the invention are separated from each other.

The photographs of Fig. 10 and Fig. 11 demonstrate that the spacer 321 formed on the lower substrate 210 is tightly attached, e.g., adhered, with the inner surface of the upper substrate 110 through the high molecular weight layer 331 before the upper substrate 110 and the lower substrate 210 are separated from each other.

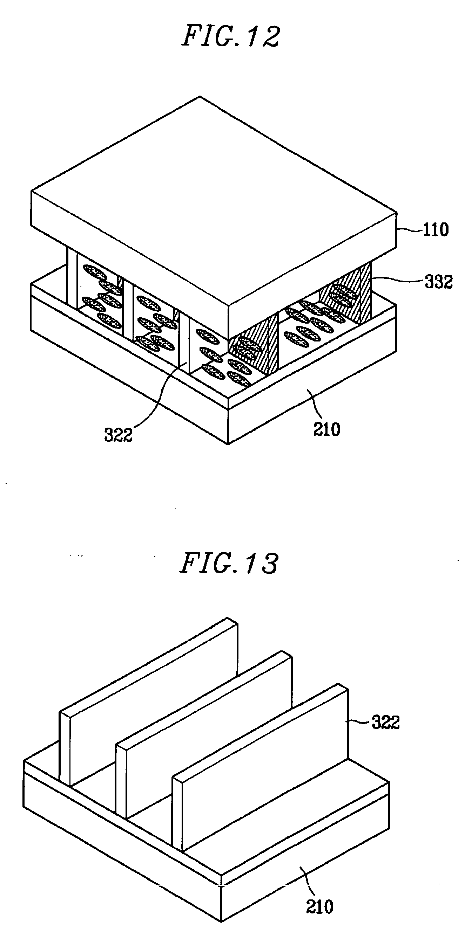

Fig. 12 is a perspective view of an LCD according to the second embodiment of the invention. Fig. 13 is a perspective view showing the condition of a spacer arranged on a common electrode panel used in the LCD shown in Fig. 12.

Referring to Fig. 12, an upper insulating substrate 110 and a lower insulating substrate 210 with a common electrode 270 are arranged to face each other, and a plurality of bar-shaped spacers 322 are arranged parallel to each other between the upper substrate 110 and the lower substrate 210. Linearly formed high molecular weight projections 332 are crossed with the spacers 322 between the upper substrate 110 and the lower substrate 210.

According to such structure, the spacer 322 defines and maintains a substantially fixed space between the upper substrate 110 and the lower substrate 210. The space is partitioned into a plurality of regions by an intersection of the spacers 322 and the high molecular weight projections 332. Each region may correspond to a pixel unit or a combination of pixel units. The regions are filled or inserted with a nematic liquid crystal.

The spacers 322 are formed via a photolithography process. For example, after the lower substrate 210 is coated with a photoresist, the photoresist-coated substrate is selectively exposed to UV light via a predetermined photo-mask and the exposed photoresist is then developed, thereby forming a spacer pattern. The spacers 322 may be made of various materials such as an elastic polymer and are not limited to being formed with photoresist.

The upper substrate 110, the spacers 322, and the lower substrate 210 are attached with the high molecular weight projections 332. The high molecular weight projections 332 are obtained by polymerizing a monomer through UV irradiation using a mask. For example, a monomer such as Norland optical adhesive (NOA-65) which is a product of Norland Co., etc., is combined with the liquid crystal 3, and the mixture is filled or inserted in the regions partitioned by the spacers 322. The upper substrate 110 is then transferred onto the spacers 322, and UV light is irradiated upon the upper substrate 110 via a predetermined mask. The monomer begins to polymerize with the UV irradiation while being anisotropically phase-separated from the liquid crystal 3. The phase-separated polymer forms projections 332 between the upper substrate 110 and the lower substrate 210.

TFTs, a pixel electrode, a common electrode, etc., are arranged on the upper substrate 110 and the lower substrate 210, which is similar to the structure shown in Fig. 3.

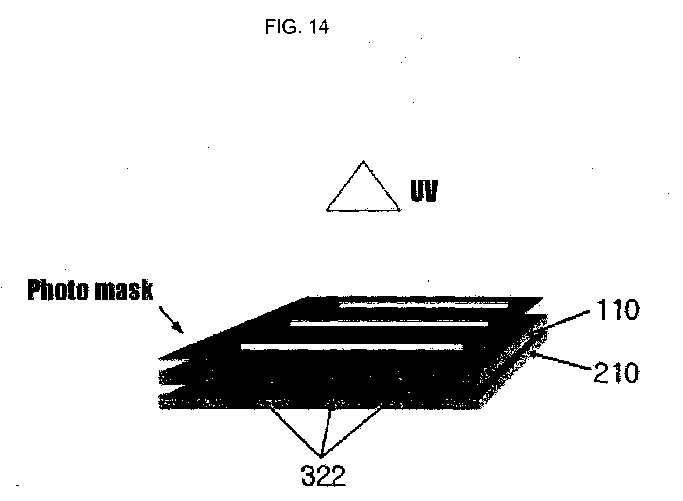

A method of assembling the upper substrate 110 and the lower substrate 210 is described below with reference to Fig. 14.

Fig. 14 shows a UV irradiation process used to produce an LCD according to the second embodiment of the invention.

A photoresist is applied or coated on the lower substrate 210 having the common electrode 270. The photoresist-coated substrate 210 is then selectively exposed to UV light via a predetermined mask and the exposed photoresist is developed, thereby forming bar-shaped spacers 322.

The spacers 322 are then coated with an aligning agent and rubbed to form an alignment layer (not shown).

A monomer that may be polymerized by UV irradiation is then mixed with liquid crystal 3. The mixture is filled in the regions partitioned by the spacers 322. For example, a particularly suitable monomer is the UV-curing epoxy NOA-65 produced by Norland Co.

The upper substrate 110 having the TFTs and the pixel electrodes 190 is arranged on the spacers 322, and UV light is irradiated upon the upper substrate 110 or the lower substrate 210. The UV irradiation may be performed using a photo-mask that includes a plurality of slits arranged substantially parallel to each other, as shown in Fig. 14. The UV-irradiated monomer begins to polymerize with phase separation from the liquid crystal. The phase-separated polymer adheres between the upper substrate 110 and the lower substrate 210, thereby forming substantially linearly formed high molecular weight projections 332.

The high molecular weight projections 332 function as a reliable adhesive that attaches, e.g., adheres, the spacers 322 and the lower substrate 210 with the upper substrate 110.

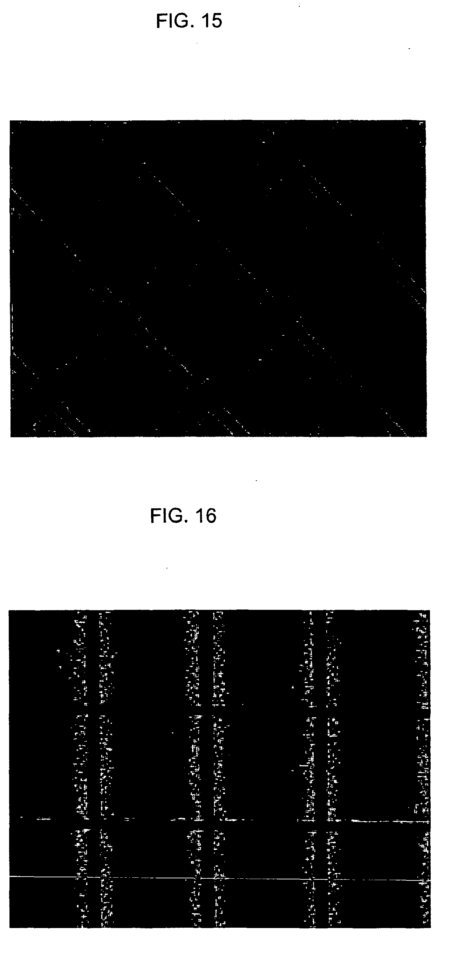

Fig. 15 is a photograph taken when the LCD shown in Fig. 12 is in a white state. Fig. 16 is a photograph taken when the LCD of Fig. 12 is in a black state.

These photographs demonstrate that the LCD of the second embodiment may display gray scale as well as black and white.

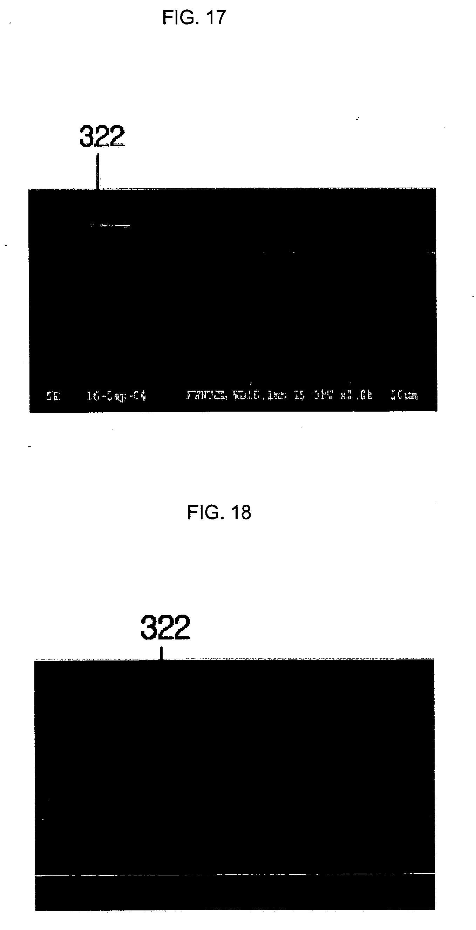

Fig. 17 and Fig. 18 are photographs taken after two insulating substrates used in an LCD according to the second embodiment of the invention are separated from each other.

The photographs of Fig. 17 and Fig. 18 demonstrate that the spacers 322 arranged on the lower substrate 210 are tightly attached, e.g., adhered, with the inner surface of the upper substrate 110 by the high molecular weight projections 332 before the upper substrate 110 and the lower substrate 210 are separated from each other.

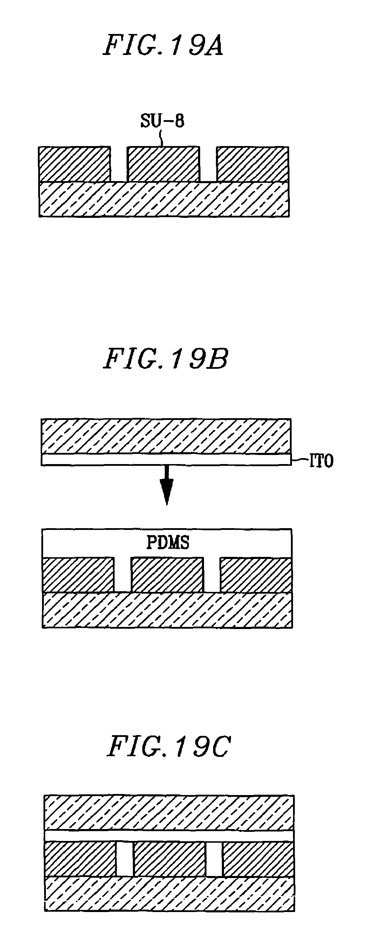

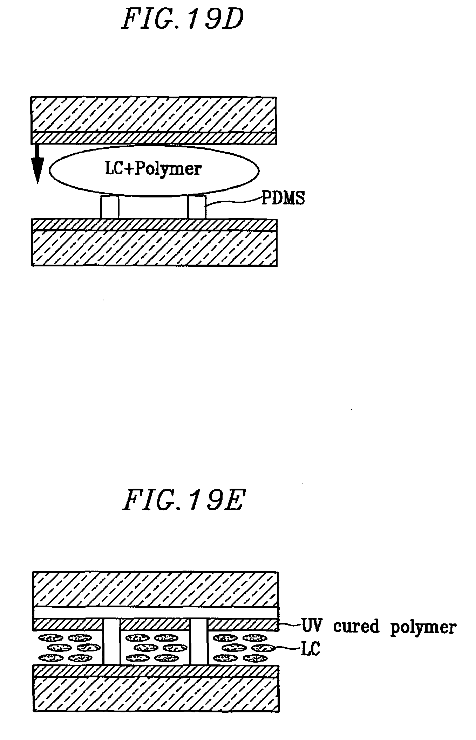

Fig. 19A, 19B, 19C, 19D, and Fig. 19E are schematic cross-sectional views showing process operations for manufacturing an LCD according to the second embodiment of the invention. In particular, these figures show process operations to form the spacers 322 using an elastic polymer.

As shown in Fig. 19A, a photoresist, such as SU-8, is applied or coated on the lower substrate 210. The photoresist-coated substrate 210 is then selectively exposed to UV light via a predetermined photo-mask and the exposed photoresist is developed, thereby forming a stamp for imprinting a spacer pattern.

As shown in Fig. 19B, a monomer of an elastic polymer, for example, PDMS [poly(dimethylsiloxane)], is applied or coated on the lower substrate 210 with the stamp. The ITO-deposited substrate 110 is then disposed thereon.

As shown in Fig. 19C, two facing insulating substrates, e.g., the upper insulating substrate 110 and the lower insulating substrate 210, are heated at approximately 100° for about 10 minutes, while being pressurized. After such heating, the frame is removed from the upper substrate 110 and the lower substrate 210. As a result, bar-shaped spacers 322 made of PDMS remain on the lower substrate 210.

As shown in Fig. 19D, an aligning agent is applied or coated on the spacers 322 by a spin coating method and rubbed to form an alignment layer. After the alignment layer is formed, the liquid crystal 3 is mixed with a monomer that can be polymerized by UV irradiation. Specifically, in this embodiment, E7 nematic liquid crystal, manufactured by Merck Industrial Chemicals, and UV epoxy NOA-65, manufactured by Norland Co., were mixed at a ratio of about 95:5. The mixture is then inserted or filled in the regions formed by the spacers 322. The ITO deposited upper substrate 110 is then arranged thereon.

As shown in Fig. 19E, UV light is then irradiated upon the upper substrate 110 or the lower substrate 210 to polymerize the monomer, resulting in the formation of high molecular weight projections 332 between the upper substrate 110 and the lower substrate 210. In manufacturing samples used for experimental testing, UV light having a wavelength of about 350nm was used, and 200W Xenon lamps were used as sources of the UV light.

A stamping technique using the stamp to imprint the spacers, as discussed above, may reduce manufacturing cost by simplifying the spacer formation process.

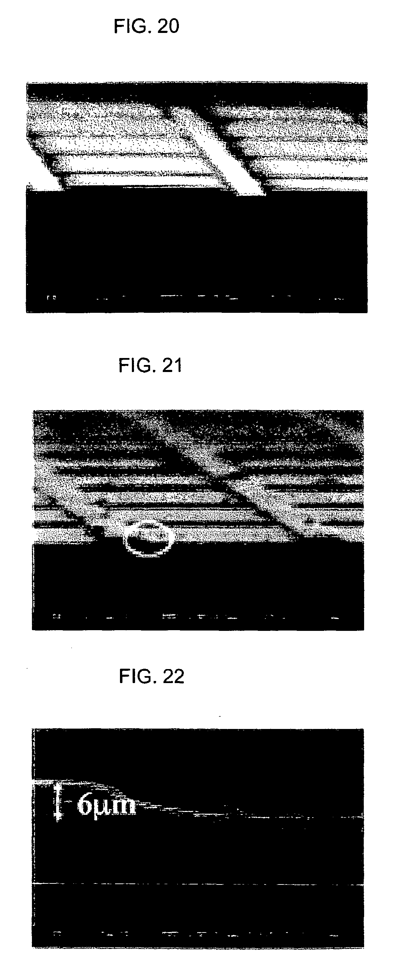

Fig. 20 shows a SEM photograph of a stamp applicable to the process operations shown in Figs. 19A, 19B, 19C, 19D, and Fig. 19E and Fig. 21 shows a SEM photograph of a spacer formed using the stamp of Fig. 20. Fig. 22 is an enlarged cross-sectional view of a portion represented as a circle shown in Fig. 21.

The photographs of Figs. 20, 21 and 22 demonstrate that the spacers formed using the stamping technique are also substantially uniform.

In the second embodiment of the invention shown in Figs. 19A, 19B, 19C, 19D and 19E, the stamp is formed by performing the photoresist deposition, exposure, and developing operations, and then the spacers are formed by applying or coating the monomer of elastic polymer, such as PDMS, on the stamp. Alternately, PDMS may be used for the formation of the stamp (though a photoetching process, etc.) and the photoresist may be used to form the spacers.

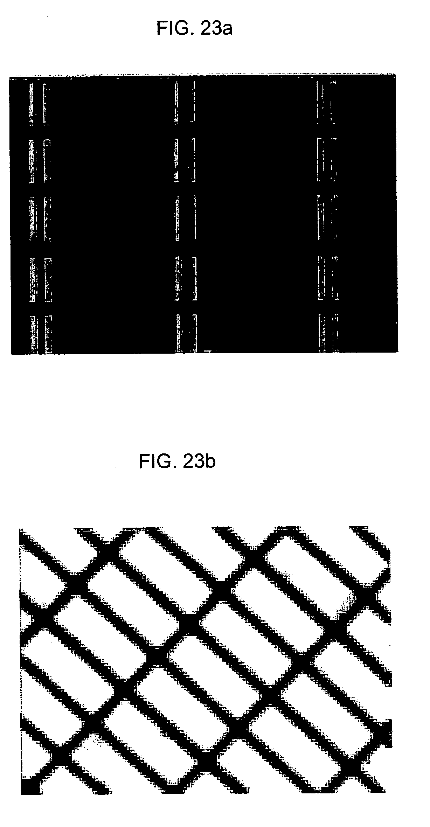

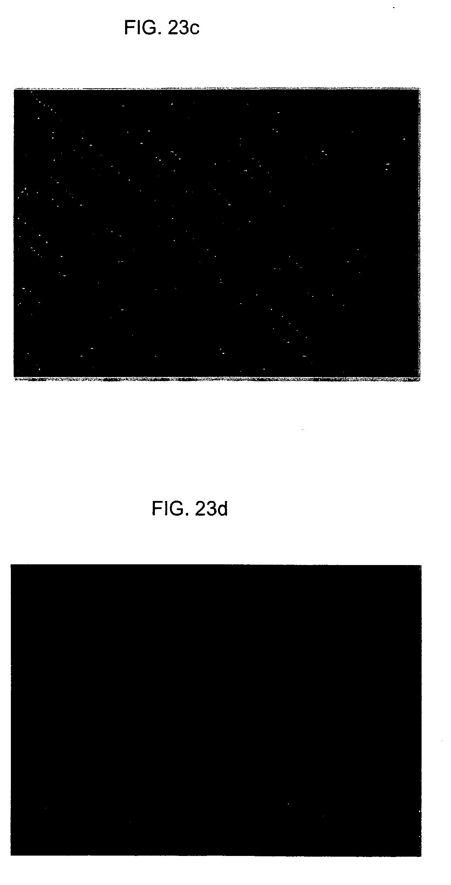

Fig. 23A and Fig. 23B are photographs taken when the LCD, manufactured through the process operations shown in Figs. 19A, 19B, 19C, 19D and 19E, is in black state and white state, respectively. Fig. 23C and Fig. 23D are photographs taken when the LCD, manufactured through the process operations shown in Figs. 19A, 19B, 19C, 19D and 19E is in a medium gray state in response to gray voltages of 3V and 6V.

Fig. 23A and Fig. 23B demonstrate that the LCD adopting the stamping method can effectively display gray scales. Although some light leakage may result, as shown in Fig. 23A, such a phenomenon may be substantially reduced or prevented by the black matrix.

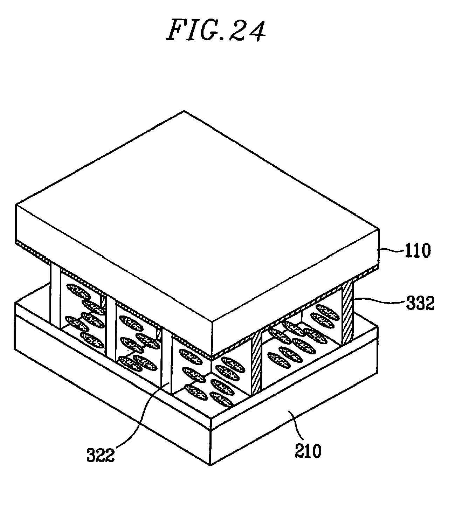

Fig. 24 is a perspective view of an LCD according to a third embodiment of the invention.

This LCD of the third embodiment further includes a high molecular weight layer formed on an inner surface of the upper substrate 110, together with the high-molecular projections 332, compared with the structure shown in Fig. 12.

Such a structure is obtained by performing UV irradiation multiple times, e.g., twice. For example, the first UV irradiation is performed to form the high molecular weight projections 332 using a photo-mask with slits, and the second UV irradiation is performed to separate the remaining monomer from the liquid crystal after the formation of the high molecular weight projections 332.

As described above, the monomer is mixed with the liquid crystal prior to the photo-polymerization. After mixing, UV irradiation is performed to form the high molecular weight layer. This high molecular weight layer attaches or adheres the spacer to the substrates. In such a structured flexible LCD, no substrate-lifting phenomenon occurs and the cell gap may be maintained substantially uniform when it is in a flexible state, e.g., folded or rolled. In addition, this invention is applicable to a roll-to-roll process since dispersion of the spacer is not required.

It will be apparent to those skilled in the art that various modifications and variation can be made in the present invention without departing from the spirit or scope of the invention. Thus, it is intended that the present invention cover the modifications and variations of this invention provided they come within the scope of the appended claims and their equivalents.

## Claims

1. A liquid crystal display (LCD), comprising:
a first insulating substrate;
a second insulating substrate arranged opposite to the first insulating substrate;
a liquid crystal layer arranged between the first insulating substrate and the second insulating substrate;
a spacer that forms and maintains a substantially uniform gap between the first insulating substrate and the second insulating substrate; and
a high molecular weight layer formed on a surface of the second insulating substrate that connects the spacer with the second insulating substrate.

2. The LCD of claim 1, further comprising:
a first electrode arranged on the first insulating substrate; and
a second electrode arranged on the second insulating substrate.

3. The LCD of claim 1, wherein the spacer is a lattice-like shape.

4. The LCD of claim 3, wherein spacer partitions the liquid crystal layer into a plurality of regions.

5. The LCD of claim 1, wherein the high molecular weight layer comprises a phase-separated polymer that is obtained by irradiating light upon a combination of a UV-curing monomer and liquid crystal that is provided in the cell gap.

6. The LCD of claim 5, wherein the light used for the phase separation of the monomer and the liquid crystal is ultraviolet (UV) light.

7. The LCD of claim 6, wherein the monomer is a UV-curing epoxy NOA-65 manufactured by Norland Co.

8. The LCD of claim 2, further comprising:
a thin film transistor arranged on the first insulating substrate and coupled with the first electrode.

9. The LCD of claim 2, further comprising:
another thin film transistor arranged on the second insulating substrate and coupled with the second electrode.

10. The LCD of claim 2, wherein the spacer comprises a plurality of bars, and wherein a plurality of high molecular weight projections are arranged substantially linear and cross the spacer.

11. The LCD of claim 1, wherein the spacer comprises an elastic polymer material.

12. The LCD of claim 1, wherein the spacer comprises a PDMS [poly(dimethylsiloxane)] material.

13. A liquid crystal display (LCD), comprising:
a first insulating substrate;
a second insulating substrate arranged opposite to the first insulating substrate;
a liquid crystal layer arranged between the first insulating substrate and the second insulating substrate;
a spacer that forms and maintains a substantially uniform gap between the first insulating substrate and the second insulating substrate; and
a high molecular weight projection that is arranged between the first insulating substrate and the second insulating substrate.

14. The LCD of claim 13, wherein the spacer comprises a plurality of bars and the high molecular weight projection is substantially linear and crosses the spacer.

15. The LCD of claim 13, further comprising:
a first electrode arranged on the first insulating substrate; and
a second electrode arranged on the second insulating substrate.

16. The LCD of claim 13, wherein the spacer and the high molecular weight projection divide the liquid crystal layer into a plurality of regions.

17. The LCD of claim 14, wherein the high molecular weight projections are comprised of a phase-separated polymer obtained by irradiating light to a mixture of a UV-curing monomer and liquid crystal provided in the ceii gap.

18. The LCD of claim 17, wherein the light used for the phase separation of the monomer and the liquid crystal is ultraviolet light.

19. The LCD of claim 18, wherein the monomer is UV-curing epoxy NOA-65 manufactured by Norland Co.

20. The LCD of claim 15, further comprising a first thin film transistor that is formed on the first insulating substrate, while being connected to the first electrode.

21. The LCD of claim 15, further comprising a second thin film transistor that is formed on the second insulating substrate, while being connected to the second electrode.

22. The LCD of claim 13, wherein the spacer is comprised of an elastic polymer.

23. The LCD of claim 22, wherein the spacer is comprised of PDMS [poly(dimethylsiloxane)].

24. A method of manufacturing a liquid crystal display, comprising:
forming a first electrode on a first insulating substrate;
forming a spacer on the first electrode;
applying a mixture of a polymerizable monomer and liquid crystalline material on the first electrode with the fixed spacer;
arranging a second insulating substrate on the fixed spacer;
phase-separating the polymerizable monomer from the liquid crystalline material during polymerization; and
attaching the phase-separated polymer to a surface of the second insulating substrate such that the spacer and the second insulating substrate are attached thereto.

25. The method of claim 24, wherein the fixed spacer contacts a second electrode formed on a surface of the second insulating substrate after the second insulating substrate is arranged on the fixed spacer.

26. The method of claim 24, further comprising:
applying an alignment material on the spacer and rubbing the coating material thereon to form an alignment layer before the first electrode is formed on the first insulating substrate.

27. The method of claim 24, wherein forming the alignment layer comprises :
applying a photoresist on the first electrode;
exposing the photoresist to light via a mask; and
developing the exposed photoresist.

28. The method of claim 24, wherein forming the spacer on the first electrode comprises:
forming a stamp for imprinting a spacer;
applying an elastic polymer on the stamp;
arranging the first insulating substrate with the first electrode on the elastic polymer; and
simultaneously heating and applying pressure to the first insulating substrate that is arranged on the elastic polymer.

29. The method of claim 28, wherein forming the stamp for imprinting the spacer comprises:
coating an SU-8 photoresist on the first electrode;
exposing the SU-8 photoresist to light via a mask; and
developing the exposed SU-8 photoresist.

30. The method of claim 24, wherein phase-separating the polymerizable monomer from the liquid crystalline material during polymerization is performed by directing UV irradiation at the mixture of the monomer and the liquid crystalline material from the exterior surface of the second insulating substrate.

31. The method of claim 30, wherein the UV irradiation is performed through a mask.
